# EUROPEAN PATENT APPLICATION

(11) **EP 4 663 742 A1**
(43) Date of publication of application: **17.12.2025**
(21) Application number: 24182255.0
(22) Date of filing: 14.06.2024
(51) Int. Cl.: C12M 1/12, C12M 1/42

(54) **DUAL-SIDED MULTI-CELL CO-CULTURE SYSTEM AND METHOD OF CULTURING CELLS**

(71) Applicant: Taipei Medical University, Taipei City 110 (TW)
(72) Inventor: TSENG, How, 110301 TAIPEI CITY (TW)
(74) Representative: Lavoix

(57) **Abstract**

A dual-sided multi-cell co-culture system and a method of culturing cells are provided. A dual-sided multi-cell co-culture system includes a first culture tank, a second culture tank, a membrane assembly, a first culture plate and a second culture plate. The membrane assembly is configured to be removably mounted to the first culture tank and configured to be removably mounted to the first culture tank. The first culture plate has at least one well, wherein the at least one well of the first culture plate is configured to receive the first culture tank equipped with the membrane assembly. The second culture plate has at least one well, wherein the at least one well of the second culture plate is configured to receive the second culture tank equipped with the membrane assembly.

## Description

### BACKGROUND

### 1. Field of the Disclosure

The instant disclosure relates to, amongst other things, a dual-sided multi-cell co-culture system and a method of culturing different groups of cells.

### 2. Description of Related Art

In the field of cell culture, traditional methods often involve the use of single-layer cell culture systems, which are generally limited to cultivating one type of cell at a time. These systems typically employ culture dishes or flasks made from materials such as polystyrene, which provide a surface for cell attachment and growth. While effective for basic cell culture, these systems face significant limitations when it comes to simulating more complex biological environments.

One major challenge in conventional cell culture techniques is the inability to effectively co-culture multiple cell types that require distinct microenvironments. For instance, anaerobic bacteria require oxygen-free conditions, whereas many mammalian cells, including epithelial and endothelial cells, require oxygenated environments. Creating a system that can maintain these differing conditions simultaneously has been a complex and costly endeavor.

Additionally, current cell culture systems often require extensive handling and manipulation of cultures, which increases the risk of contamination and the variability of experimental results. This is particularly problematic in applications that require high precision and reproducibility, such as drug testing and disease modeling.

### SUMMARY

According to one example embodiment of the instant disclosure, a dual-sided multi-cell co-culture apparatus includes: a first culture tank, a second culture tank and a membrane assembly. The first culture tank includes a main body being substantially cylindrical and having a first end and a second end opposite to the first end, a first opening at the first end of the main body; and a second opening at the second end of the main body. The second culture tank includes a main body being substantially cylindrical and having a first end and a second end opposite to the first end, a first opening at the first end of the main body; and a second opening at the second end of the main body. The membrane assembly is configured to be removably mounted at the first end of the main body of the first culture tank. When the membrane assembly is mounted at the first end of the main body of the first culture tank, the membrane assembly is configured to substantially seal the first opening of the main body of the first culture tank. The membrane assembly is configured to be removably mounted at the first end of the main body of the second culture tank. When the membrane assembly is mounted at the first end of the main body of the second culture tank, the membrane assembly is configured to substantially seal the first opening of the main body of the second culture tank.

According to another example embodiment of the instant disclosure, a dual-sided multi-cell co-culture system includes a first culture tank, a second culture tank, a membrane assembly, a first culture plate and a second culture plate. The membrane assembly is configured to be removably mounted to the first culture tank and configured to be removably mounted to the first culture tank. The first culture plate has at least one well, wherein the at least one well of the first culture plate is configured to receive the first culture tank. When the first culture tank is mounted with the membrane assembly and received in the at least one well of the first culture plate, a first surface of the membrane assembly faces an inner space of the first culture tank and a second surface of the membrane assembly faces a bottom of the at least one well of the first culture plate. The second culture plate has at least one well, wherein the at least one well of the second culture plate is configured to receive the second culture tank. When the second culture tank is mounted with the membrane assembly and received in the at least one well of the second culture plate, the second surface of the membrane assembly faces an inner space of the second culture tank and the first surface of the membrane assembly faces a bottom of the at least one well of the second culture plate.

According to another example embodiment of the instant disclosure, a method of co-culturing cells includes: equipping a membrane assembly with a first culture tank, wherein a first surface faces an inner space of the first culture tank; arranging the first culture tank with the membrane assembly in a well of a first culture plate; cultivating a first group of cells on the first surface of the membrane assembly; removing the first culture tank with the membrane assembly from the well of the first culture plate; separating the membrane assembly from the first culture tank; equipping the membrane assembly with a second culture tank, wherein a second surface, which is opposite to the first surface, faces an inner space of the second culture tank; arranging the second culture tank with the membrane assembly in a well of a second culture plate; and cultivating a second group of cells on the second surface of the membrane assembly.

In order to further understanding of the instant disclosure, the following embodiments are provided along with illustrations to facilitate appreciation of the instant disclosure; however, the appended drawings are merely provided for reference and illustration, and do not limit the scope of the instant disclosure.

### BRIEF DESCRIPTION OF THE DRAWINGS

FIG. 1 is a schematic view of a dual-sided multi-cell co-culture apparatus in accordance with an embodiment of the instant disclosure.
FIG. 2A is a schematic view of a membrane assembly of a dual-sided multi-cell co-culture apparatus in accordance with an embodiment of the instant disclosure.
FIG. 2B illustrates a schematic cross-sectional view of a membrane assembly of a dual-sided multi-cell co-culture apparatus in accordance with an embodiment of the instant disclosure.
FIG. 3A is a schematic view of a first culture tank of a dual-sided multi-cell co-culture apparatus in accordance with an embodiment of the instant disclosure.
FIG. 3B is another schematic view of a first culture tank of a dual-sided multi-cell co-culture apparatus in accordance with an embodiment of the instant disclosure.
FIG. 3C illustrates a schematic cross-sectional view of a first culture tank of a dual-sided multi-cell co-culture apparatus in accordance with an embodiment of the instant disclosure.
FIG. 4 illustrates a schematic cross-sectional view of a first culture tank equipped with a membrane assembly in accordance with an embodiment of the instant disclosure.
FIG. 5A, FIG. 5B, FIG. 5C are schematic illustrations of operations of separating a membrane assembly from a first culture tank in accordance with an embodiment of the instant disclosure.
FIG. 6A is a schematic view of a second culture tank of a dual-sided multi-cell co-culture apparatus in accordance with an embodiment of the instant disclosure.
FIG. 6B is another schematic view of a second culture tank of a dual-sided multi-cell co-culture apparatus in accordance with an embodiment of the instant disclosure.
FIG. 6C illustrates a schematic cross-sectional view of a second culture tank of a dual-sided multi-cell co-culture apparatus in accordance with an embodiment of the instant disclosure.
FIG. 7 illustrates a schematic cross-sectional view of a second culture tank equipped with a membrane assembly in accordance with an embodiment of the instant disclosure.
FIG. 8A, FIG. 8B, FIG. 8C, FIG. 8D are schematic illustrations of operations of equipping a membrane assembly with a second culture tank in accordance with an embodiment of the instant disclosure.
FIG. 9A, FIG. 9B, FIG. 9C are schematic illustrations of operations of separating a membrane assembly from a second culture tank in accordance with an embodiment of the instant disclosure.
FIG. 10A is a schematic view of a first culture plate in accordance with an embodiment of the instant disclosure.
FIG. 10B is a schematic view showing a first culture tank with a membrane assembly received in one of wells of the first culture plate in accordance with an embodiment of the instant disclosure.
FIG. 11A is a schematic view of a second culture plate in accordance with an embodiment of the instant disclosure.
FIG. 11B is a schematic view of a second culture tank with a membrane assembly received in one of wells of the second culture plate in accordance with an embodiment of the instant disclosure.
FIG. 12A, FIG. 12B. FIG. 12C, FIG. 12D, FIG. 12E, FIG. 12F, FIG. 12G and FIG 12H illustrate a method of culturing cells in accordance with an embodiment of the instant disclosure.

### DETAILED DESCRIPTION

The following disclosure provides for many different embodiments, or examples, for implementing different features of the provided subject matter. Specific examples of components and arrangements are described below to explain certain aspects of the present disclosure. These are, of course, merely examples and are not intended to be limiting. For example, the formation of a first feature over or on a second feature in the description that follows may include embodiments in which the first and second features are formed or disposed in direct contact, and may also include embodiments in which additional features are formed or disposed between the first and second features, such that the first and second features are not in direct contact. In addition, the present disclosure may repeat reference numerals and/or letters in the various examples. This repetition is for the purpose of simplicity and clarity and does not in itself dictate a relationship between the various embodiments and/or configurations discussed.

As used herein, spatially relative terms, such as "beneath," "below," "above," "over," "on," "upper," "lower," "left," "right," "vertical," "horizontal," "side" and the like, may be used herein for ease of description to describe one element or feature's relationship to another element(s) or feature(s) as illustrated in the figures. The spatially relative terms are intended to encompass different orientations of the device in use or operation in addition to the orientation depicted in the figures. The device may be otherwise oriented (rotated 90 degrees or at other orientations) and the spatially relative descriptors used herein may likewise be interpreted accordingly. It should be understood that when an element is referred to as being "connected to" or "coupled to" another element, it may be directly connected to or coupled to the other element, or intervening elements may be present.

Present disclosure provides a novel method and system for co-culturing different groups of cells. It is used for simulating in vitro living tissues, such as those involved in transdermal absorption, general blood vessels, and brain blood vessels. One of the critical innovations of this system is the ability to cultivate different cell groups on both sides of the membrane. This dual-sided approach allows for the simultaneous cultivation of multiple cell types, which can interact through the membrane while maintaining their unique environmental conditions.

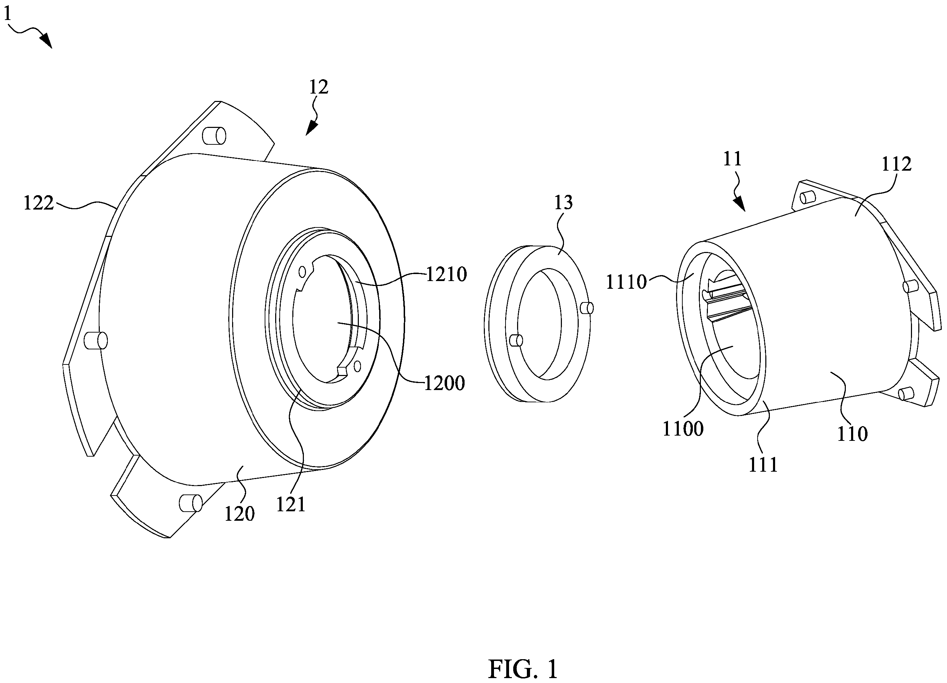

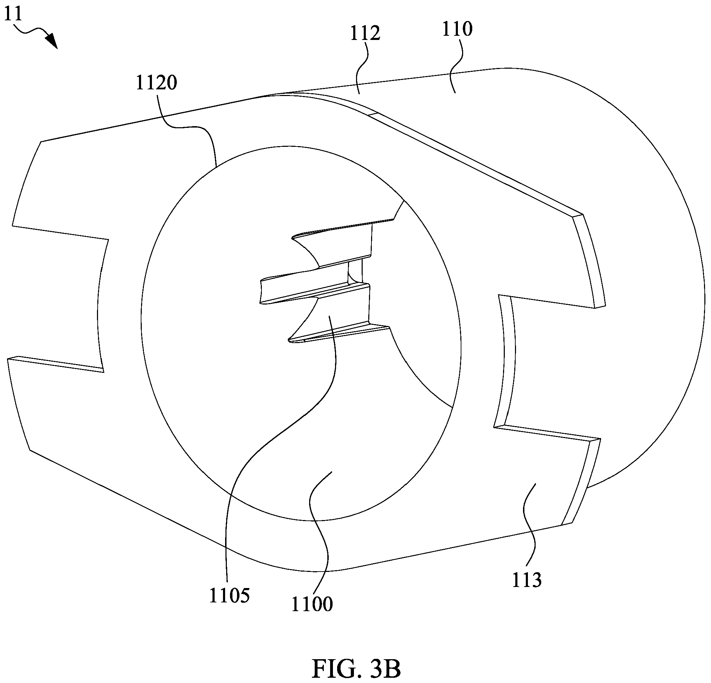

FIG. 1 is a schematic view of a dual-sided multi-cell co-culture apparatus 1 in accordance with an embodiment of the instant disclosure. As shown in FIG. 1, the dual-sided multi-cell co-culture apparatus 1 may include a first culture tank 11, a second culture tank 12, and a membrane assembly 13. Referring to Fig. 1, the first culture tank 11 may include a hollow and substantially cylindrical main body 110, and the main body 110 may include a first end 111 and a second end 112 opposite to the first end 111. In some embodiments of the present disclosure, the main body 110 is tapered from the second end 112 to the first end 111. Further, the first end 111 of the main body 110 has a first opening 1110, and the second end 112 of the main body 110 has a second opening 1120 (see FIG. 3B), and both the first opening 1110 and the second opening 1120 are in fluid communication with an inner space 1100 of the main body 110. This design allows for efficient fluid exchange and cell growth within the tank, providing an optimal environment for cellular interactions and nutrient flow. The tapered structure also aids in the easy removal and addition of culture media, enhancing the overall usability of the apparatus.

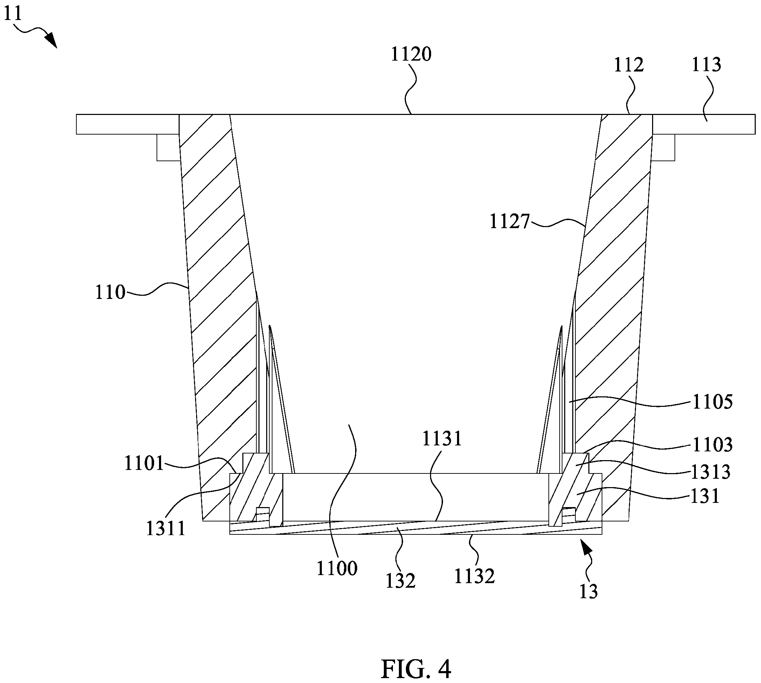

Moreover, the second culture tank 12 may include a hollow and substantially cylindrical main body 120, and the main body 120 may include a first end 121 and a second end 122 opposite to the first end 121. In some embodiments of the present disclosure, the main body 120 is tapered from the second end 122 to the first end 121. Further, the first end 121 of the main body 120 has a first opening 1210, and the second end 122 of the main body 120 has a second opening 1220, and both the first opening 1210 and the second opening 1220 (see FIG. 4B) are in fluid communication with an inner space 1200 of the main body 120. This configuration ensures that the second culture tank 12 provides similar advantages as the first culture tank 11, including efficient fluid dynamics and enhanced cellular environment. The cylindrical and tapered design of both tanks ensures that they can be easily manufactured and maintained, promoting consistency in experimental conditions.

The membrane assembly 13 is configured to be mounted to the first end 111 of the main body 110 of the first culture tank 11. That is, the membrane assembly 13 is configured to be equipped with the first culture tank 11. When the membrane assembly 13 is mounted to the first end 111 of the main body 110 of the first culture tank 11, the membrane assembly 13 is configured to substantially seal the first opening 1110 of the main body 110 of the first culture tank 11. The membrane assembly 13 is configured to be mounted to the first end 121 of the main body 120 of the second culture tank 12. That is, the membrane assembly 13 is configured to be equipped with the second culture tank 12. When the membrane assembly 13 is mounted to the first end 121 of the main body 120 of the second culture tank 12, the membrane assembly 13 is configured to substantially seal the first opening 1210 of the main body 120 of the second culture tank 12. In addition, the side of the membrane assembly 13 that faces the inner space 1100 of the main body 110 when the membrane assembly 13 is equipped with the first culture tank 11 is different from the side of the membrane assembly 13 that faces the inner space 1200 of the main body 120 when the membrane assembly 13 is equipped with the second culture tank 12.

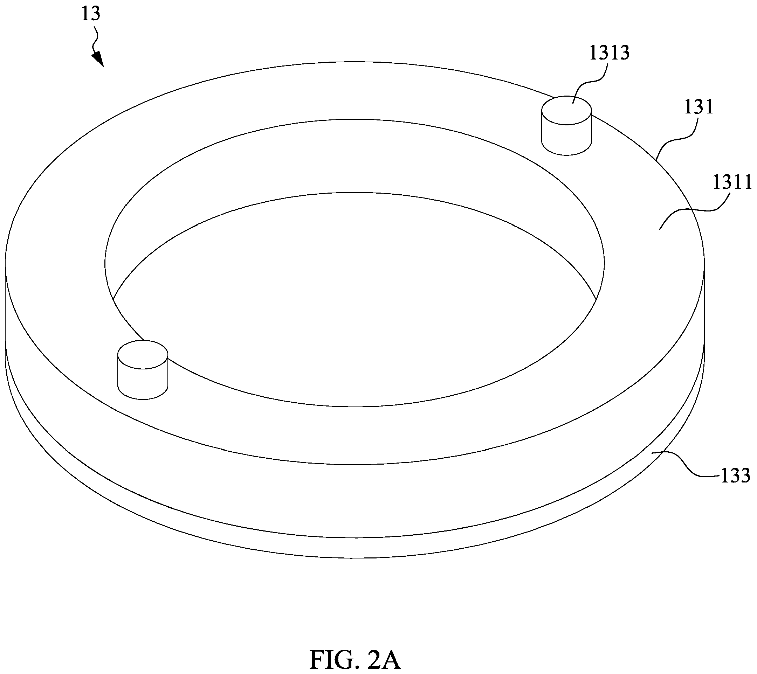

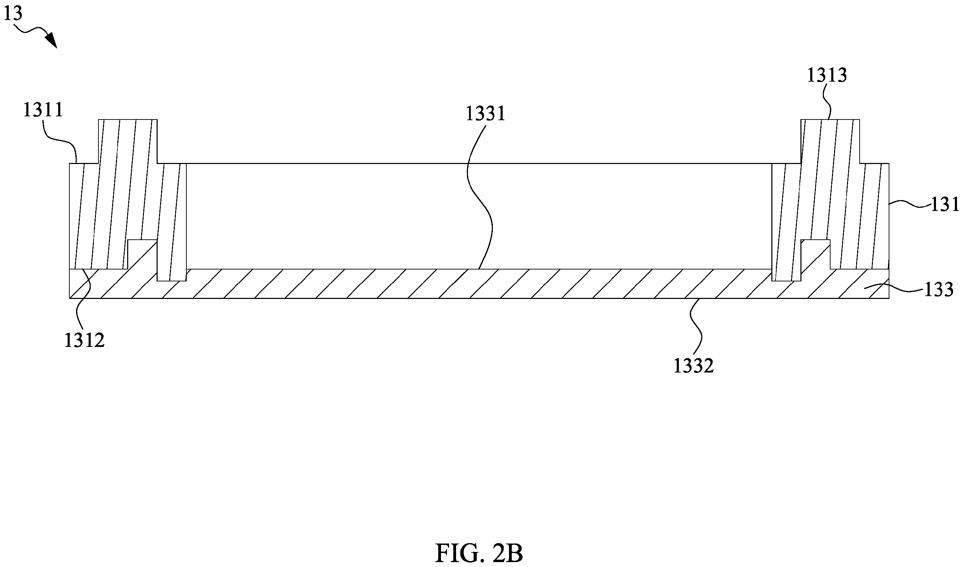

FIG. 2A is a schematic view of the membrane assembly 13 of the dual-sided multi-cell co-culture apparatus 1 in accordance with an embodiment of the instant disclosure. FIG. 2B illustrates a schematic cross-sectional view of the membrane assembly 13 of the dual-sided multi-cell co-culture apparatus 1 in accordance with an embodiment of the instant disclosure. Referring to FIG. 2A and FIG. 2B, the membrane assembly 13 may include a ring 131 and a membrane 133. The ring 131 has two opposite sides 1311 and 1312. The side 1311 of the ring 131 may have two protrusions 1313, and the side 1312 of the ring 131 may be attached to the membrane 133. As shown in FIG. 2B, the membrane 133 may cover the side 1312 of the ring 131. That is, a surface 1331 of the membrane 133 may be attached to the side 1312 of the ring 131 and recessed with respect to the side 1311 of the ring 131. Moreover, a surface 1332 of the membrane 133, which is opposite the surface 1331, is substantially flat. In some embodiments of the present disclosure, the membrane 133 is joined to the ring 131 using a non-adhesive fusion method. This construction ensures a robust and leak-proof assembly, which is crucial for maintaining the integrity of the co-culture conditions. The non-adhesive fusion method also minimizes any potential chemical interference from adhesives, ensuring a more biocompatible environment for cell cultures.

The membrane 133 is configured to culture different groups of cells on two opposite surfaces 1331 and 1332 respectively. The membrane 133 may include a transparent porous membrane, and the transparent porous membrane is made of a material such as PET, PC, PTFE, PVDF, etc. The pore size of the transparent porous membrane may be 400 nm, 1 micron, 3 microns, or 8 microns. These varying pore sizes allow for selective permeability, supporting the growth and interaction of diverse cell types. Moreover, one or more biopolymers may be grafted onto both surfaces 1331 and 1332 of the membrane 133 to adjust the stiffness of the substrate surface that the cells or tissues contact. In some embodiments of the present disclosure, the one or more biopolymers may be grafted onto both surfaces 1331 and 1332 of the membrane 133 by performing a plasma treatment. The one or more biopolymers may include gamma-PGA, hyaluronic acid, collagen, chitin, chitosan, fibroin, and/or poly-L-lysine. This grafting process enhances the membrane's biocompatibility and functional properties, allowing researchers to tailor the cellular microenvironment precisely.

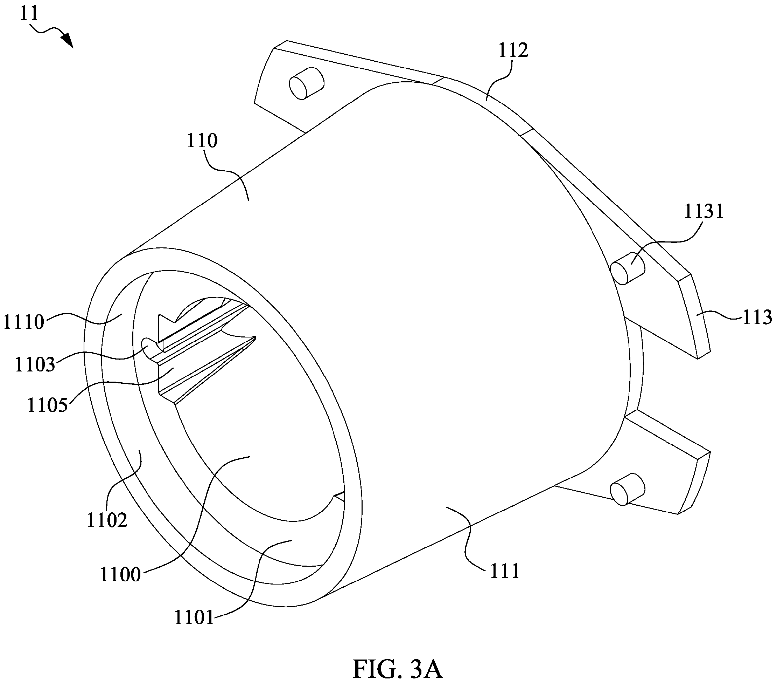

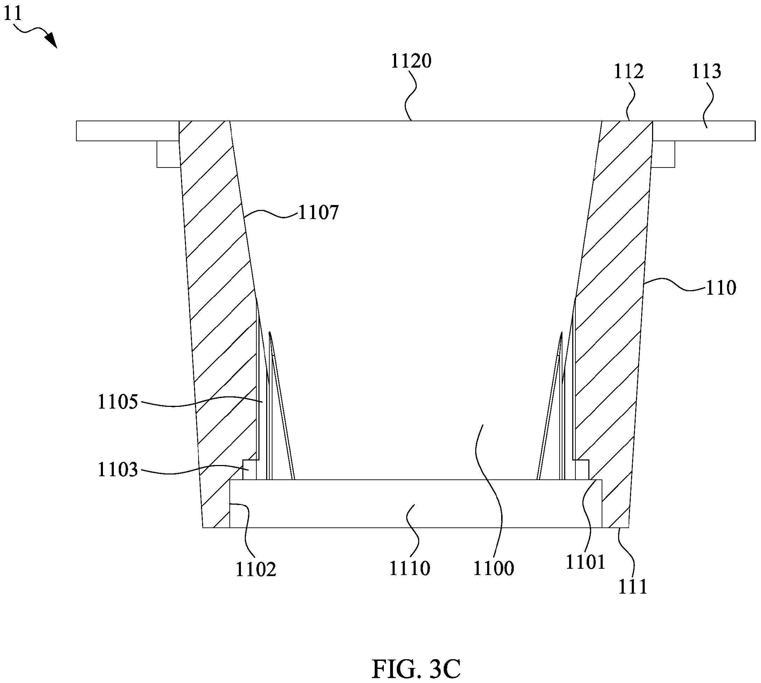

FIG. 3A is a schematic view of the first culture tank 11 of the dual-sided multi-cell co-culture apparatus 1 in accordance with an embodiment of the instant disclosure. FIG. 3B is another schematic view of the first culture tank 11 of the dual-sided multi-cell co-culture apparatus 1 in accordance with an embodiment of the instant disclosure. FIG. 3C illustrates a schematic cross-sectional view of the first culture tank 11 of the dual-sided multi-cell co-culture apparatus 1 in accordance with an embodiment of the instant disclosure. As shown in FIG. 3A, FIG. 3B and FIG. 3C, the first culture tank 11 may have a hollow main body 110 which may be substantially cylindrical and tapered from the second end 112 toward the first end 111. The second end of the main body 110 may include the second opening 1120 that is in fluid communication with the inner space 1100 of the main body 110. Further, the main body 110 may include a plurality of flanges 113 adjacent to the second end 112 of the main body 110. Each of the flange 113 may include a protrusion 1131.

The first end 111 of the main body 110 may include the first opening 1110 that is in fluid communication with the inner space 1100 of the main body 110. The first opening 1110 may include an inner periphery 1102. Moreover, the inner space 1100 of the main body 110 may include a ring-shaped surface 1101 being adjacent to the first opening 1110 and connected to the inner periphery 1102. Two concaves 1103 may be formed on the ring-shaped surface 1101 and two grooves 1105 formed on an inner surface 1107 of the inner space 1100 and connected to the concaves 1103 respectively. Referring to FIG. 3A, FIG. 3B and FIG. 3C, the grooves 105 may extend substantially longitudinally from the ring-shaped surface 1101 toward the inner space 1100 of the main body 110.

Further referring to FIG. 4, when the membrane assembly 13 is mounted to the first end 111 of the main body 110 of the first culture tank 11, the membrane assembly 13 may be received in the first opening 1110. The ring 131 of the membrane assembly 13 may tightly match the inner periphery 1102 of the first opening 1110, ensuring a secure and leak-proof fit. Moreover, the side 1311 of the ring 131 may abut against the ring-shaped surface 1101, and the protrusions 1313 of the ring 131 may be inserted into the concaves 1103, respectively, allowing the membrane assembly 13 to engage firmly with the first end 111 of the main body 110 of the first culture tank 11. This precise alignment and secure fitting are crucial for maintaining the integrity of the co-culture environment, preventing contamination, and ensuring consistent experimental conditions. In addition, the surface 1331 of the membrane 133 may face the inner space 1100 of the main body 110, providing a suitable surface for cell attachment and growth. The surface 1332 of the membrane 133 may face away from the inner space 1100 of the main body 110, allowing for the separation of different cell types or compartments.

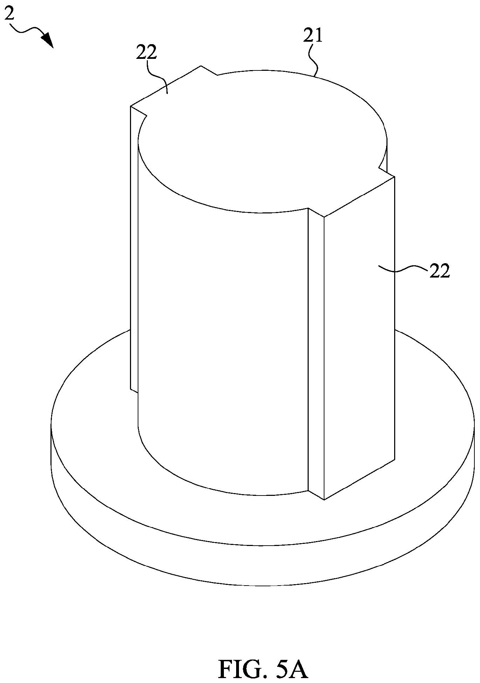

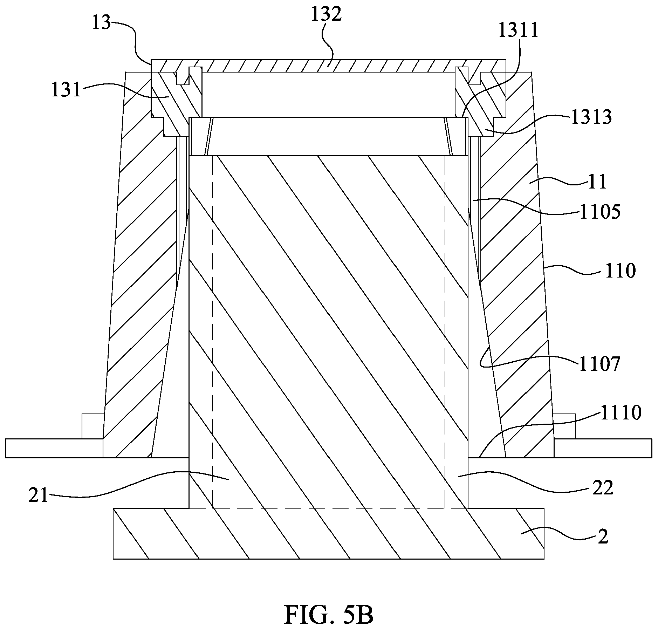

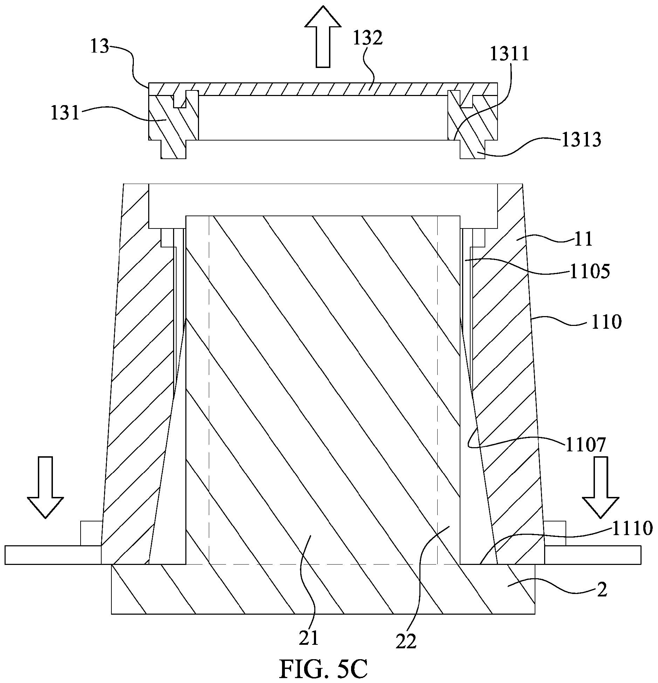

FIG. 5A, FIG. 5B, FIG. 5C are schematic illustrations of operations of separating the membrane assembly 13 from the first culture tank 11 in accordance with an embodiment of the instant disclosure. Referring to FIG. 5A, a tool 2 is provided. The tool 2 may include a cylinder 21, and the cylinder 21 may include two flanges 22. The flanges 22 extend substantially longitudinally along the outer surface of the cylinder 21. The tool 2 may facilitate the safe and efficient removal of the membrane assembly without causing damage to either the membrane or the culture tank. The flanges 22 may ensure proper alignment and leverage during the removal process.

Referring to FIG. 5B, the cylinder 21 of the tool 2 is inserted into the inner space 1100 of the main body 110 of the first culture tank 11 from the second opening 1120. The flanges 22 of the cylinder 21 match the grooves 1105 formed on the inner surface 1107 of the inner space 1100 respectively. This alignment is crucial for the effective transmission of force and ensures that the tool engages correctly with the internal features of the culture tank. Moreover, the tops 220 of the flanges 22 contact the protrusions 1313 of the ring 131 of the membrane assembly 13, which are inserted into the concaves 1103 of the ring-shaped surface 1101 of the main body 110. This contact allows the tool to exert pressure precisely on the protrusions, facilitating their disengagement.

Referring to FIG. 5C, a user continues pressing down on the first culture tank 11 to further insert the cylinder 21 of the tool 2 towards the first opening 1110 of the main body 110. The tops 220 of the flanges 22 of the tool 2 further push against the side 1311 and/or the protrusions 1313 of the ring 131 of the membrane assembly 13, causing the protrusions 1313 to disengage from the concaves 1103 of the ring-shaped surface 1101 of the main body 110. This action effectively separates the membrane assembly 13 from the first culture tank 11. This method ensures a controlled and damage-free removal of the membrane assembly, which is essential for maintaining the integrity of the experimental setup and ensuring the reusability of the components. The design of the tool and the step-by-step process provide a reliable and repeatable method for membrane assembly removal, enhancing the practicality and user-friendliness of the dual-sided multi-cell co-culture apparatus.

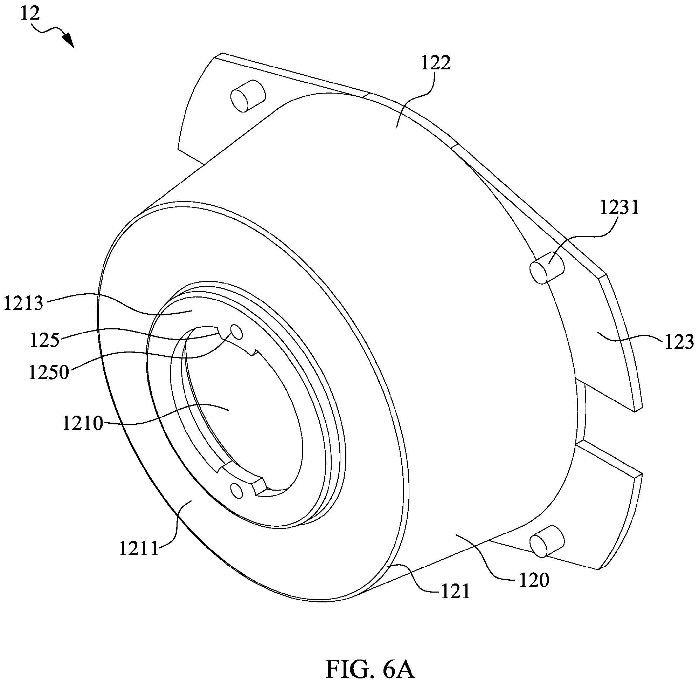

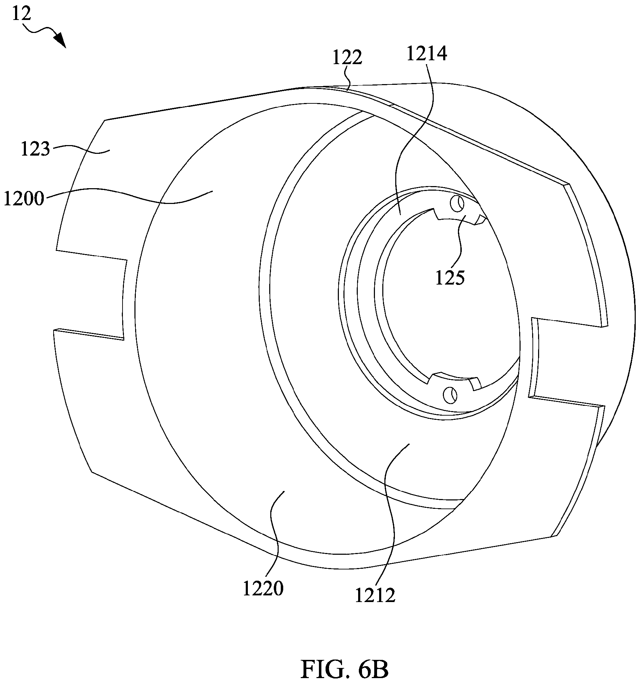

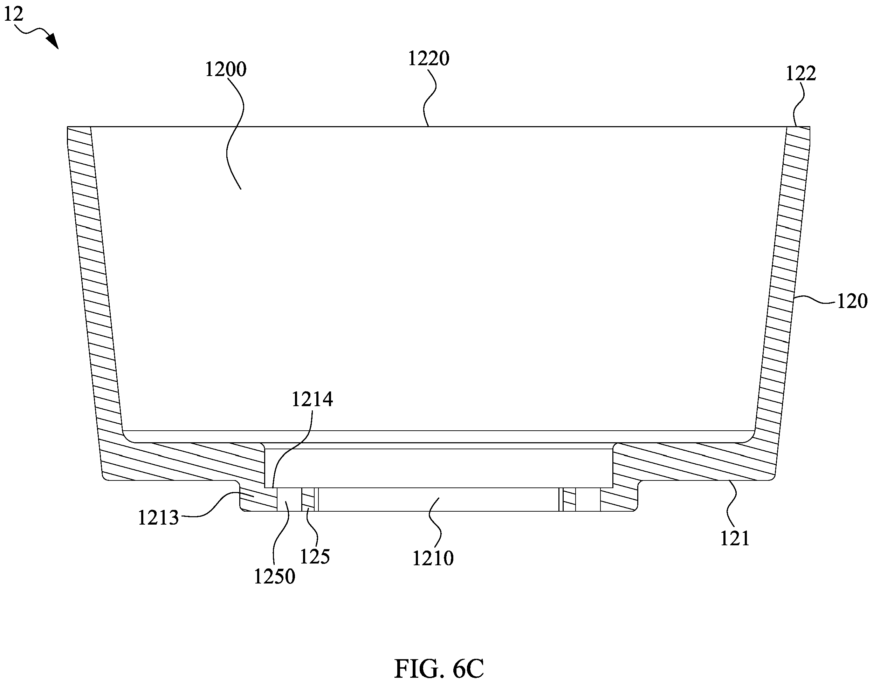

FIG. 6A is a schematic view of the second culture tank 12 of the dual-sided multi-cell co-culture apparatus 1 in accordance with an embodiment of the instant disclosure. FIG. 6B is another schematic view of the second culture tank 12 of the dual-sided multi-cell co-culture apparatus 1 in accordance with an embodiment of the instant disclosure. FIG. 6C illustrates a schematic cross-sectional view of the second culture tank 12 of the dual-sided multi-cell co-culture apparatus 1 in accordance with an embodiment of the instant disclosure. As shown in FIG. 6A, FIG. 6B and FIG. 6C, the second culture tank 12 may have a hollow main body 120 which may be substantially cylindrical and tapered from the second end 122 toward the first end 121. The second end 122 of the main body 110 may include the second opening 1220 that is in fluid communication with the inner space 1200 of the main body 120. Further, the main body 120 may include a plurality of flanges 123 adjacent to the second end 122 of the main body 120. Each of the flange 123 may include a protrusion 1231.

The first end 121 of the main body 120 may include the first opening 1210 that is in fluid communication with the inner space 1200 of the main body 120. The first end 121 of the main body 120 may include a surface 1211 facing away from the inner space 1200 of the main body 120 and a surface 1212 facing the inner space 1200 of the main body 120 and a surface 1212. The surface 1212 of the first end 121 may include a substantially ring-shaped recess 1214, wherein the recess 1214 may surround the first opening 1210. Further, the surface 1211 of the first end 121 may include a substantially ring-shaped protrusion 1213 corresponding to the recess 1214 formed on the surface 1212 and surrounding the first opening 1210. Moreover, the first opening 1210 may include two flanges 125 formed at a periphery of the first opening 1210. Each of the flange 125 may include a through hole 1250.

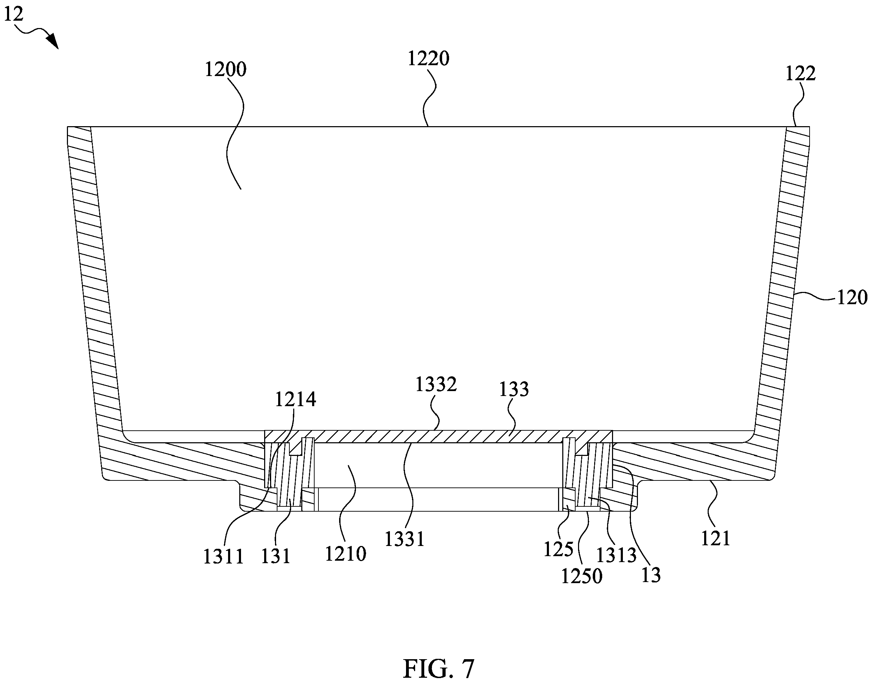

Further referring to Fig. 7, when the membrane assembly 13 is mounted to the first end 121 of the main body 120 of the second culture tank 12, the membrane assembly 13 may be received in the recess 1214 of the first end 121 and substantially aligned with the first opening 1210. The side 1311 of the ring 131 partially abuts against the recess 1214 and the flanges 125, and the protrusions 1313 of the ring 131 are inserted into the through holes 1250 of the flanges 125, respectively, allowing the membrane assembly 13 to securely engage with the first end 121 of the main body 120 of the second culture tank 12. This design ensures a stable and leak-proof connection, which is essential for maintaining distinct environments on either side of the membrane for co-culture experiments.

In addition, the surface 1332 of the membrane 133 may face the inner space 1200 of the main body 120, providing an optimal surface for cell attachment and growth within the second culture tank. Conversely, the surface 1331 of the membrane 133 may face away from the inner space 1200, which is crucial for maintaining separation between different cell types or experimental conditions.

Moreover, when the membrane assembly 13 is mounted to the first end 121 of the main body 120 of the second culture tank 12, a portion of the side 1311 of the ring 131 may be not covered by the protrusion 1213 of the surface 1211 of the first end 121. This exposed portion of the ring 131 may extends along the periphery of the first opening 1210, providing an accessible edge that facilitates easy removal and handling of the membrane assembly. This design feature ensures that the membrane can be securely fastened while also allowing for its straightforward removal when necessary, enhancing the usability and practicality of the dual-sided multi-cell co-culture apparatus.

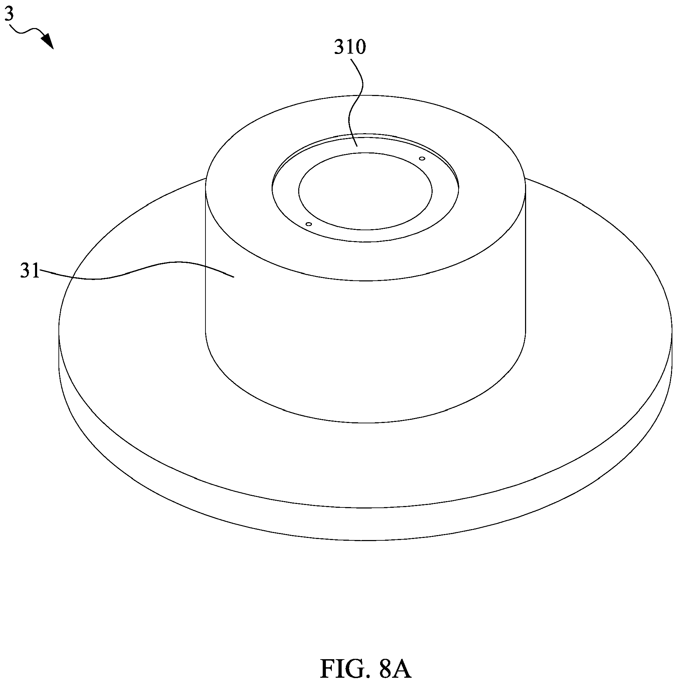

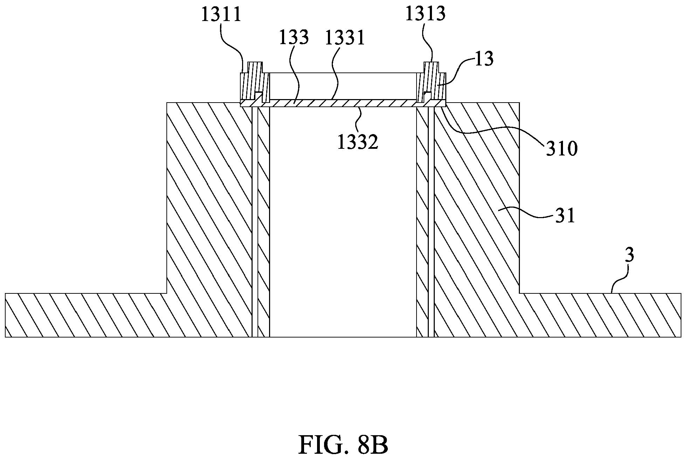

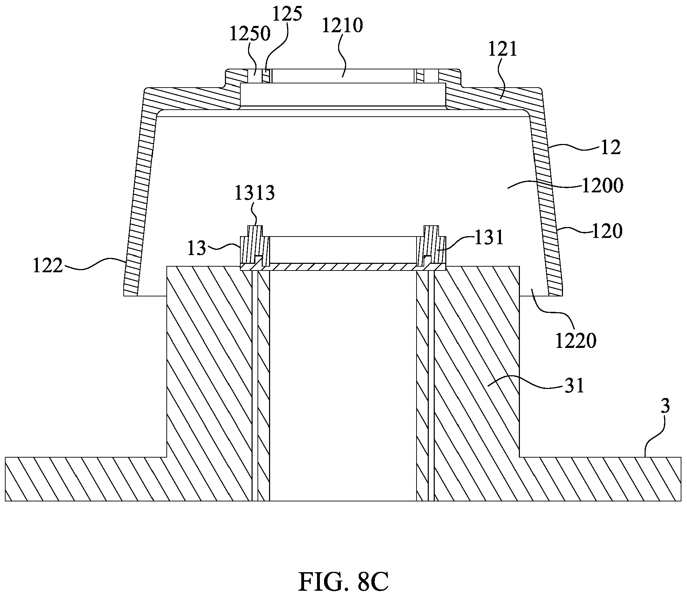

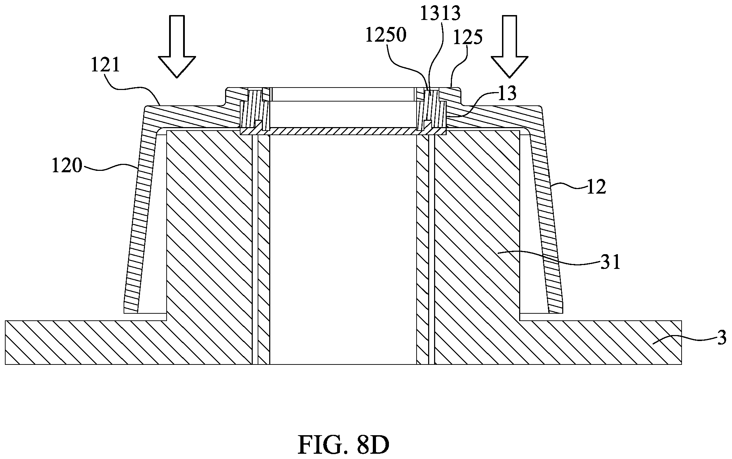

FIG. 8A, FIG. 8B, FIG. 8C, FIG. 8D are schematic illustrations of operations of equipping the membrane assembly 13 with the second culture tank 12 in accordance with an embodiment of the instant disclosure. Referring to FIG. 8A, a tool 3 is provided. The tool 3 may include a substantially hollow cylinder 31, which features a ring-shaped recess 310. This recess 310 may be formed on the top of the cylinder 31 and surround the hollow space of the cylinder 31. The tool 3 may facilitate the precise placement and secure attachment of the membrane assembly 13 to the second culture tank 12, ensuring proper alignment and minimizing the risk of damage during installation.

Referring to Fig. 8B, the membrane assembly 13 is received in the recess 310 of the cylinder 31 of the tool 3, with the side 1311 of the ring 131 and the surface 1331 of the membrane 133 facing upward. Additionally, the protrusions 1313 of the ring 131 extend beyond the recess 310 of the cylinder 31. This configuration ensures that the membrane assembly 13 is held securely in place and correctly oriented for insertion into the culture tank.

Referring to FIG. 8C, the cylinder 31 of the tool 3 is inserted into the inner space 1200 of the main body 120 of the second culture tank 12 from the second opening 1220. The membrane assembly 13 may be aligned with the first opening 1210 of the main body 120, wherein the protrusions 1313 of the ring 131 of the membrane assembly 13 may be aligned with the through holes 1250 of the flanges 125 of the first opening 1210.

Referring to Fig. 8D, a user continues pressing down on the second culture tank 12 to further insert the cylinder 31 of the tool 3 towards the first opening 1210 of the main body 120. As the user applies pressure, the protrusions 1313 of the ring 131 of the membrane assembly 13 may be pushed into the through holes 1250 of the flanges 125 of the first opening 1210. This action causes the protrusions 1313 to engage securely with the flanges 125, ensuring that the membrane assembly 13 is firmly attached to the first end 121 of the main body 120 of the second culture tank 12. This method ensures a secure and stable installation of the membrane assembly, which is crucial for maintaining the experimental conditions required for accurate and reproducible co-culture experiments.

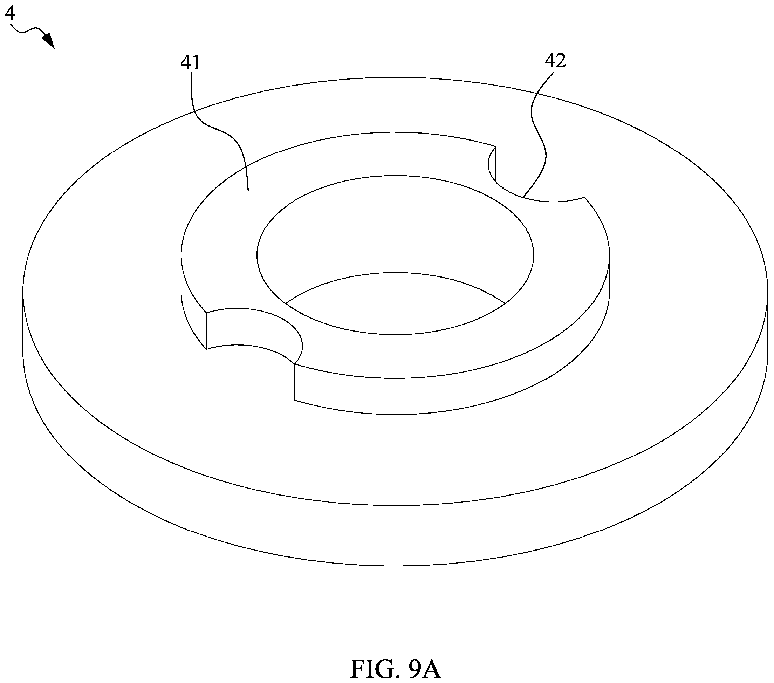

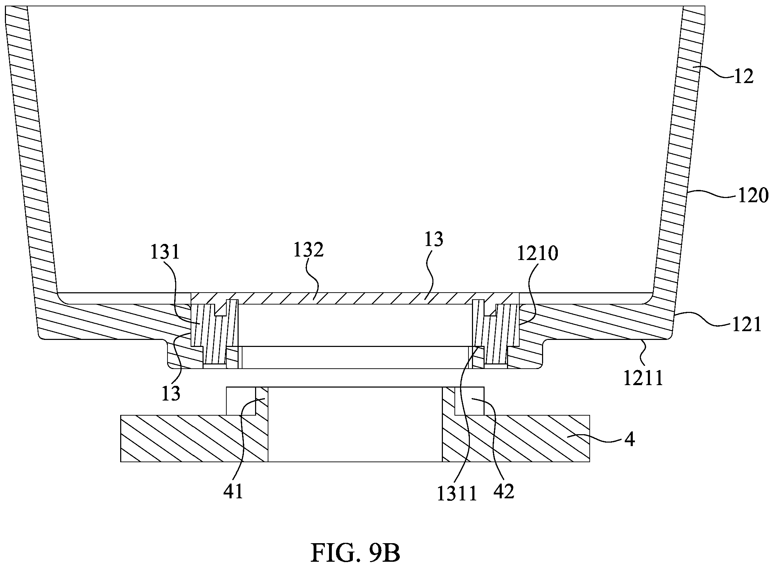

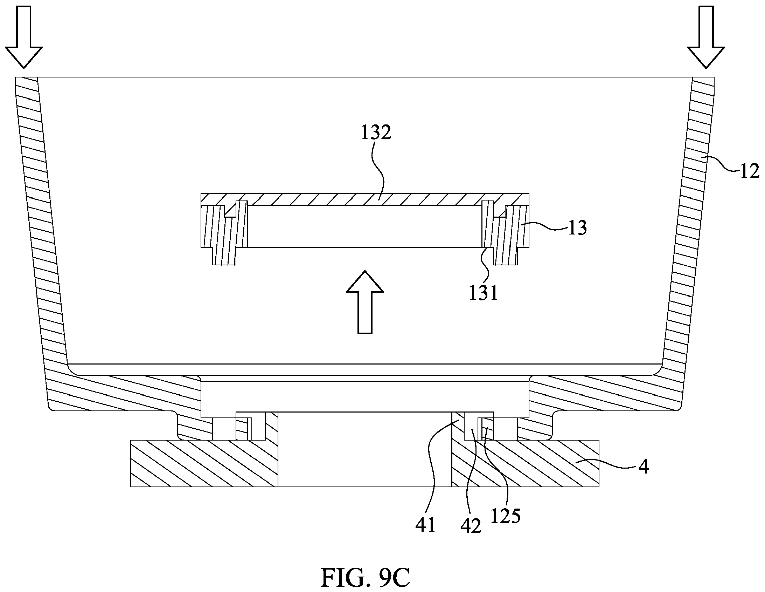

FIG. 9A, FIG. 9B, FIG. 9C are schematic illustrations of operations of separating the membrane assembly 13 from a second culture tank 12 in accordance with an embodiment of the instant disclosure. Referring to FIG. 9A, a tool 4 is provided. The tool 4 includes a hollow protrusion 41, which features two grooves 42. These grooves 42 extend substantially longitudinally along the outer surface of the protrusion 41. The tool 4 is specifically designed to facilitate the safe and efficient removal of the membrane assembly 13 from the second culture tank 12, ensuring that the membrane and tank remain undamaged during the process.

Referring to FIG. 9B, the second culture tank 12 is placed on the tool 4. The first opening 1210 of the main body 120 of the second culture tank 12 is substantially aligned with the protrusion 41. The flanges 125 of the first opening 1210 are aligned with the grooves 42 of the protrusion 41. Additionally, the portion of the ring 131 of the membrane assembly 13 that is exposed at the surface 1211 of the first end 121 of the main body 120 abuts against the top of the protrusion 41. This alignment ensures that the tool 4 is correctly positioned to exert force on the membrane assembly in a controlled manner.

Referring to Fig. 9C, a user continues pressing down on the second culture tank 12. As pressure is applied, the tops of the protrusion 41 of the tool 4 push against the ring 131 of the membrane assembly 13. This action causes the protrusions 1313 to disengage from the through holes 1250 of the flanges 125 of the first opening 1210. As the flanges 125 slip into the grooves 42 of the protrusion 41, the membrane assembly 13 is effectively detached from the second culture tank 12. This method ensures a clean and damage-free removal of the membrane assembly, maintaining the integrity of both the membrane and the culture tank for future use. The design of the tool and the step-by-step process provide a reliable and user-friendly method for disassembling the co-culture apparatus, enhancing its practicality and efficiency in laboratory settings.

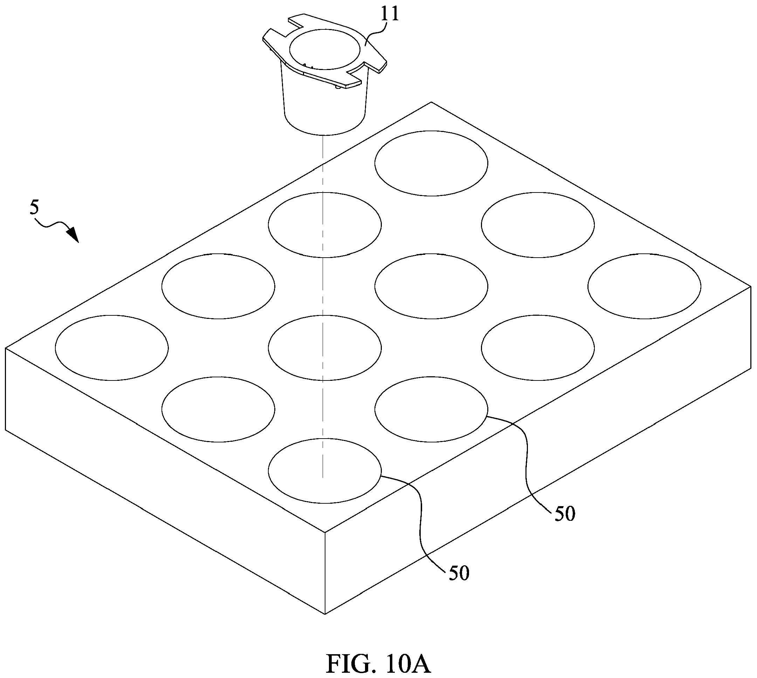

FIG. 10A is a schematic view of a first culture plate 5 in accordance with an embodiment of the instant disclosure. As shown in FIG. 10A, the first culture plate 5 includes a plurality of wells 50. In some embodiments, the first culture plate 5 includes twelve wells arranged in a matrix. Each well 50 is configured to receive a first culture tank 11. This arrangement allows for simultaneous culture of multiple samples under consistent conditions, which is essential for high-throughput experiments and comparative studies. The design of the culture plate and wells ensures that each culture tank can be securely held in place, providing a stable environment for cell growth and interaction.

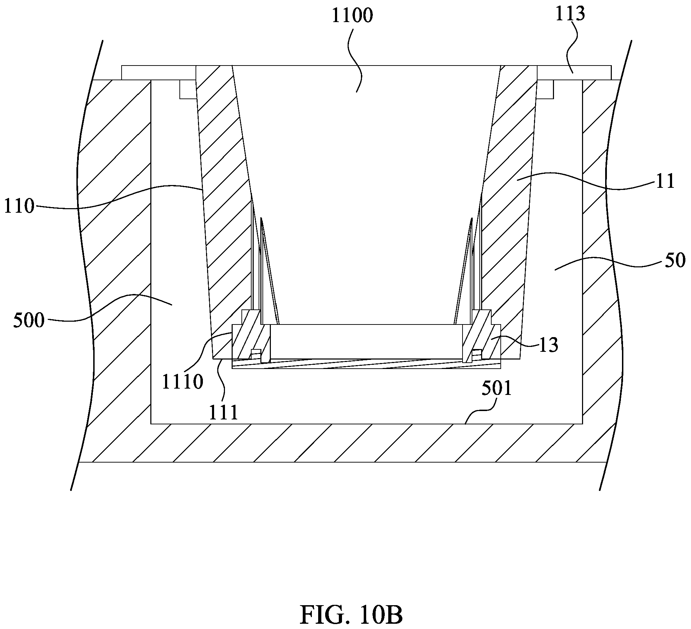

FIG. 10B shows that the first culture tank 11 with the membrane assembly 13 is received in one of the wells 50 of the first culture plate 5. Referring to Fig. 10B, the main body 110 of the first culture tank 11 is inserted into the inner space 500 of the well 50. The flanges 113 of the first culture tank 11 are supported by the upper surface of the first culture plate 5, ensuring that the membrane assembly 13 mounted to the first end 111 of the main body 110 is spaced apart from the bottom 501 of the well 50 by a certain distance. This spacing is crucial as it prevents direct contact between the membrane and the bottom of the well, which could otherwise affect cell growth and membrane function.

Further, as previously mentioned, the membrane assembly 13 is configured to substantially seal the first opening 1110 of the main body 110 of the first culture tank 11. This sealing ensures that the inner space 1100 of the main body 110 of the first culture tank 11 is isolated from the inner space 500 of the well 50 of the first culture plate 5. This separation may be vital for maintaining distinct experimental conditions within the culture tank and the well, allowing researchers to control and monitor specific environments for cell cultures. The design may also facilitate easy access to the culture medium and cells within the well, enhancing the overall usability and flexibility of the co-culture apparatus in various experimental setups.

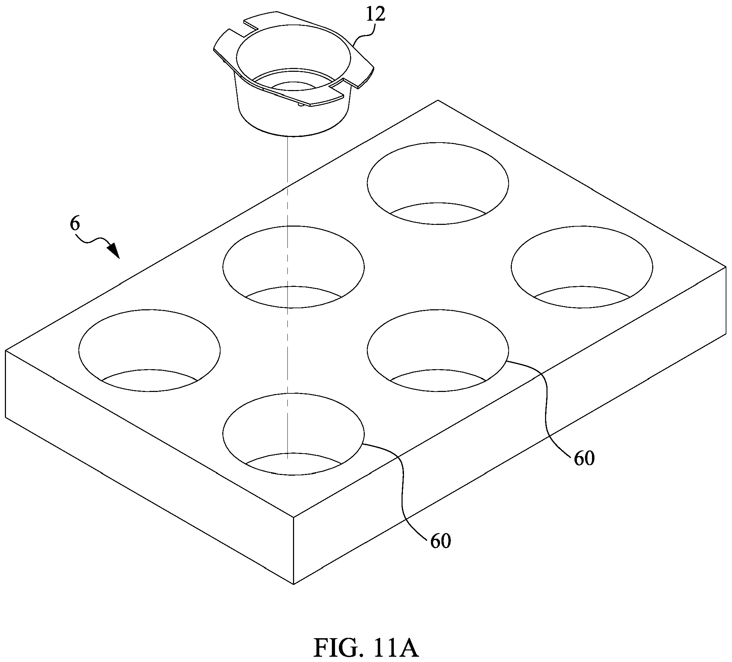

FIG. 11A is a schematic view of a first culture plate 6 in accordance with an embodiment of the instant disclosure. As shown in FIG. 11A, the first culture plate 6 includes a plurality of wells 60. In some embodiments, the first culture plate 6 includes six wells arranged in a matrix. Each well 60 is configured to receive a second culture tank 12. This design enables researchers to conduct multiple experiments simultaneously under identical conditions, facilitating comparative analyses and high-throughput screening. The arrangement of the wells ensures that each culture tank is securely positioned, providing a stable and controlled environment for cell cultures.

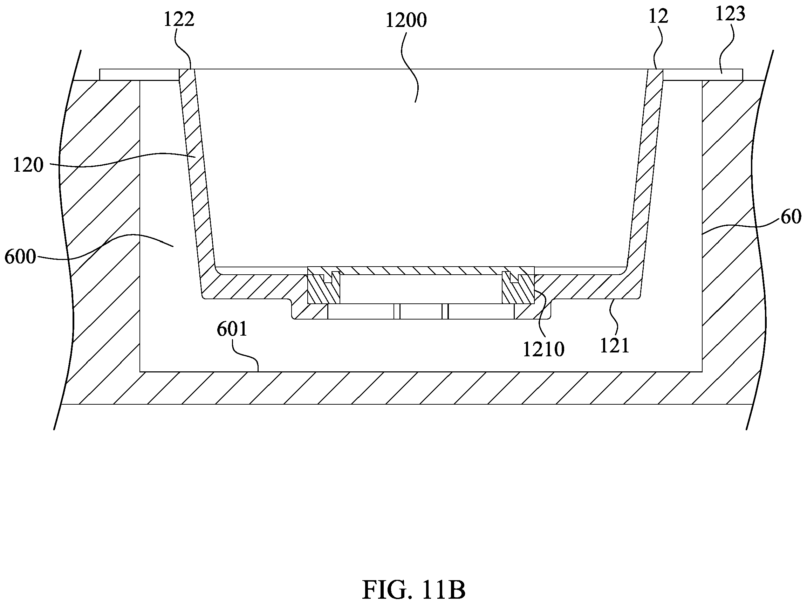

FIG. 11B shows that the second culture tank 12 with the membrane assembly 13 is received in one of the wells 60 of the second culture plate 6. Referring to Fig. 11B, the main body 120 of the second culture tank 12 is inserted into the inner space 600 of the well 60. The flanges 123 of the second culture tank 12 are supported by the upper surface of the second culture plate 6, ensuring that the membrane assembly 13 mounted to the first end 121 of the main body 120 is spaced apart from the bottom 601 of the well 60 by a certain distance. This spacing is crucial as it prevents direct contact between the membrane and the bottom of the well, which could otherwise interfere with the cell culture process and affect the results of the experiment.

Furthermore, as previously mentioned, the membrane assembly 13 is configured to substantially seal the first opening 1210 of the main body 120 of the second culture tank 12. This sealing ensures that the inner space 1200 of the main body 120 of the second culture tank 12 is isolated from the inner space 600 of the well 60 of the second culture plate 6. This separation may be essential for maintaining distinct experimental conditions within the culture tank and the well, allowing researchers to control and monitor specific environments for cell cultures. The design may also facilitate easy access to the culture medium and cells within the well, enhancing the overall usability and flexibility of the co-culture apparatus in various experimental setups.

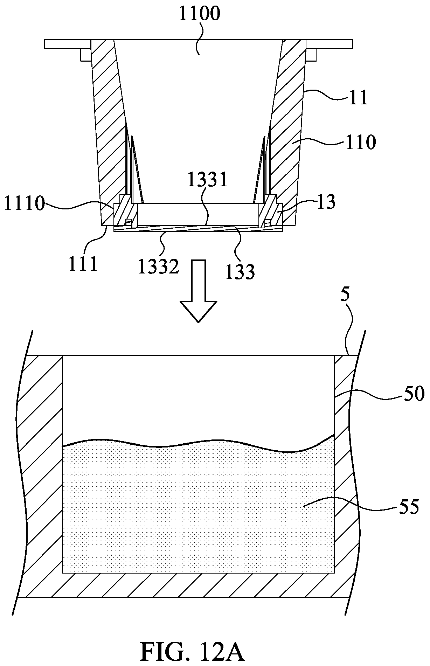

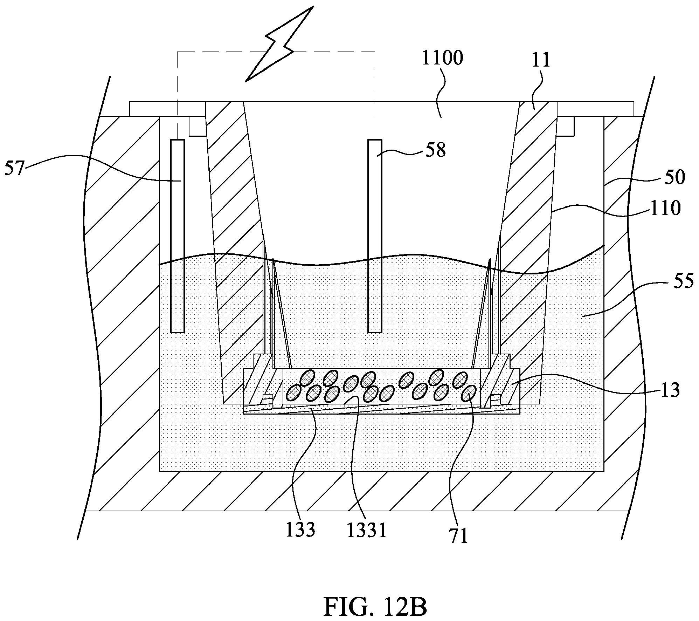

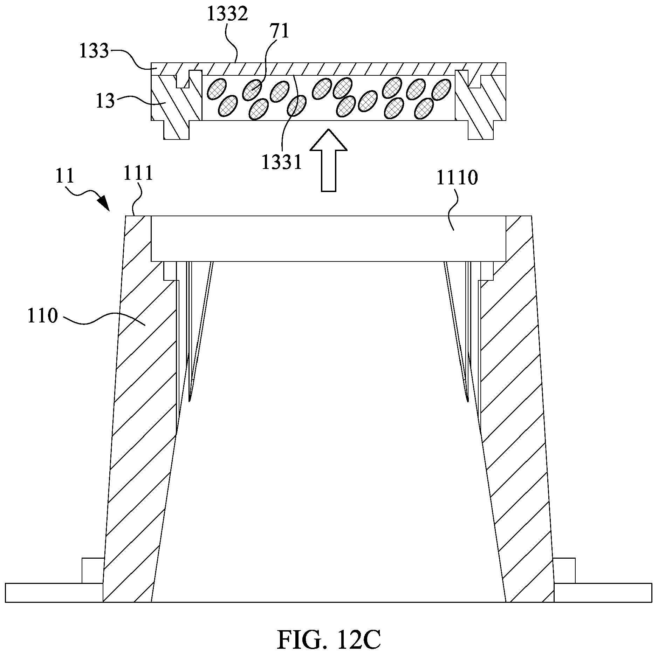

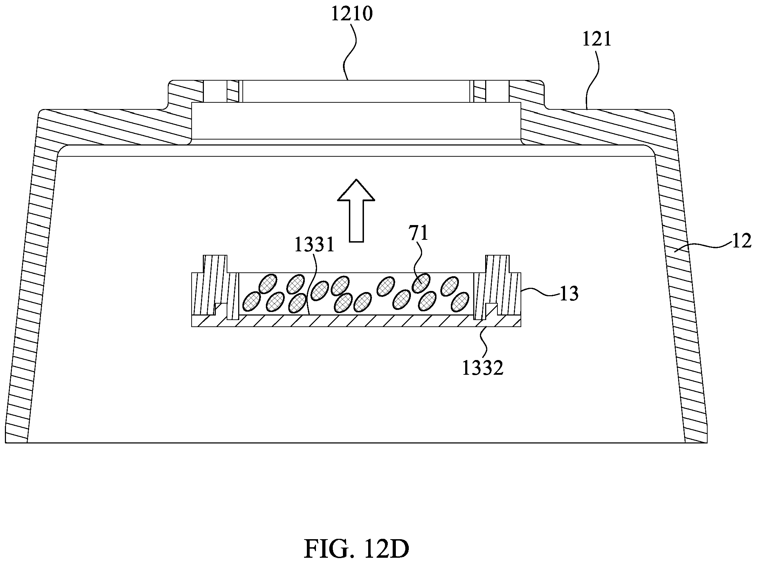

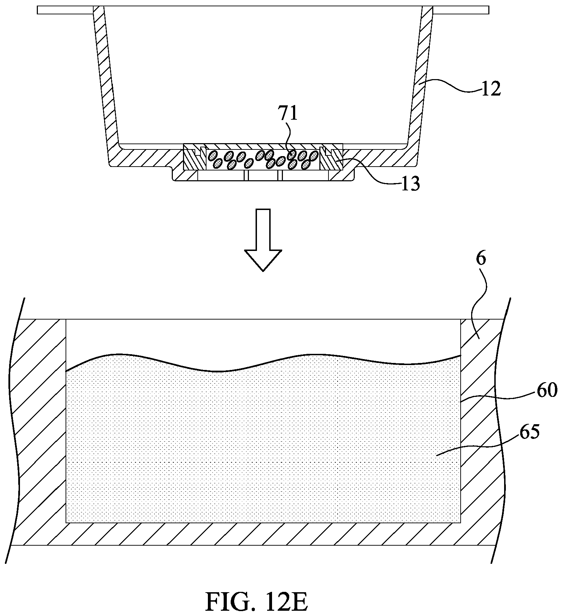

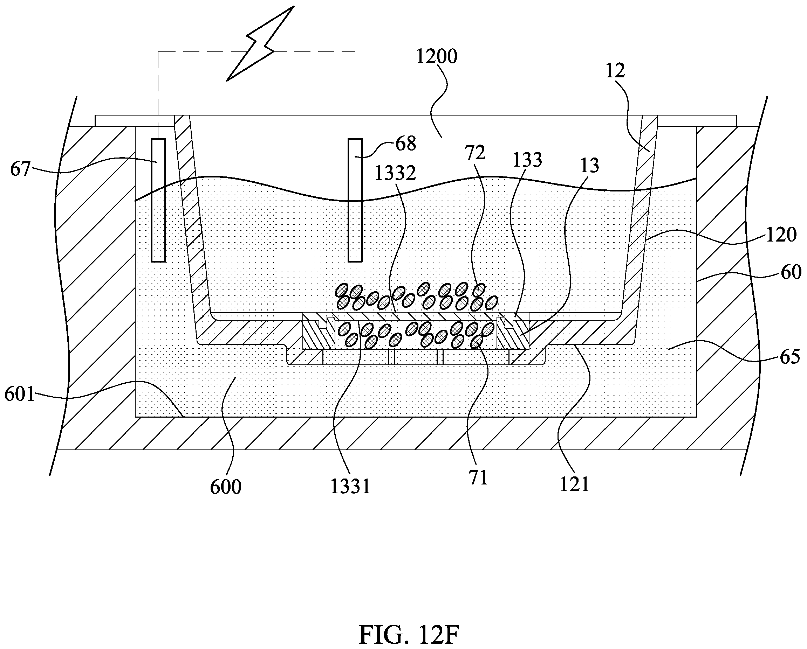

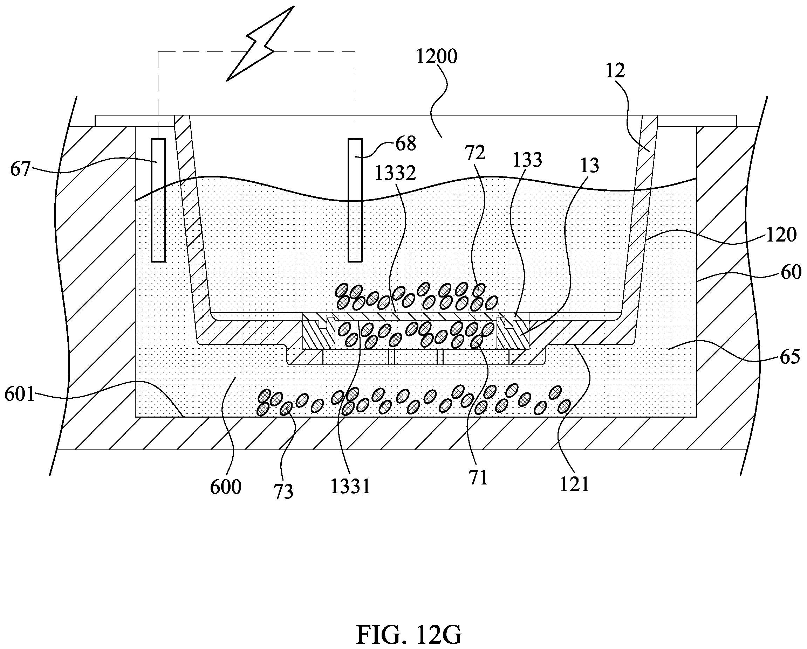

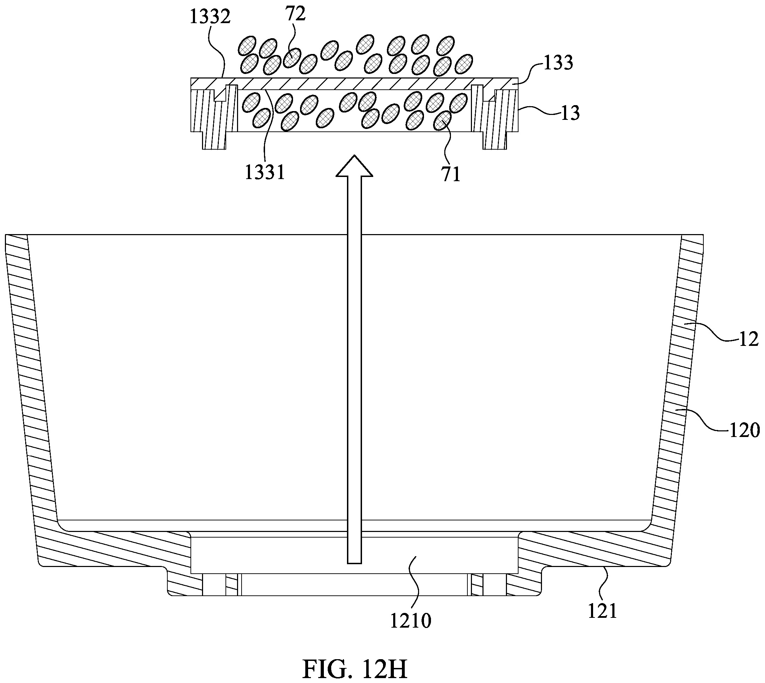

FIG. 12A, FIG. 12B. FIG. 12C, FIG. 12D, FIG. 12E, FIG. 12F, FIG. 12G and FIG. 12H illustrate a method of culturing cells in accordance with an embodiment of the instant disclosure. Referring to FIG. 12A, the first culture tank 11 with the membrane assembly 13 is received in one of wells 50 of the first culture plate 5. In some embodiments of the present disclosure, the twelve first culture tanks 11 equipped with membrane assemblies 13 may be placed individually in the twelve wells 50 of the first culture plate 5. As above mentioned, when the membrane assembly 13 is mounted to the first end 111 of the main body 110 of the first culture tank 11, the membrane assembly 13 may substantially seal the first opening 1110 and the surface 1331 of the membrane 133 of the membrane assembly 13 may face the inner space 1100 of the main body 110 of the first culture tank 11. In addition, the well 50 may be filled with cell culture medium 55.

Referring to FIG. 12B, a first group of cells 71 is cultivated in the first culture tank 11. Referring to FIG. 12B, the main body 110 of the first culture tank 11 is inserted into the well 50 of the first culture plate 5. The first end 111 of the main body 110 of the first culture tank 11 is submerged in the cell culture medium 55, causing the entire membrane assembly 13 to also be submerged in the cell culture medium 55. The first group of cells 71 may be cultivated in the inner space 1100 of the main body 110 of the first culture tank 11. The first group of cells 71 may be grown on the surface 1331 of the membrane 133 of the membrane assembly 13. That is, the first group of cells 71 may be cultivated in a horizontal state. In some embodiments of the present disclosure, the first group of cells 71 includes one or more types of cells.

Moreover, the trans-epithelial electrical resistance (TEER) may be monitored during the culture process. As shown in FIG. 12B, the electrodes 57, 58 may be respectively arranged in the inner space 1100 of the main body 110 of the first culture tank 11 and the inner space 500 of the well 50 of the first culture plate 5 so as to measure an electrical resistance across the membrane assembly 13.

Referring to FIG. 12C, after the first group of cells 71 has grown to cover the surface 1331 of the membrane 133 of the membrane assembly 13, the first culture tank 11 may be removed from the well 50 of the first culture plate 5, and the membrane assembly 13 may be detached from the first end 111 of the main body 110 of the first culture tank 11. In some embodiments of the present disclosure, the membrane assembly 13 is detached from the first end 111 of the main body 110 of the first culture tank 11 using the tool 2 and/or the method shown in FIG. 5A, FIG. 5B, and FIG. 5C.

Referring to FIG. 12D, the membrane assembly 13 is mounted to the first end 121 of the main body 120 of the second culture tank 12. In some embodiments of the present disclosure, the membrane assembly 13 is mounted to the first end 121 of the main body 120 of the second culture tank 12 using the tool 3 and/or the method shown in FIG. 8A, FIG. 8B, FIG. 8C and FIG. 8D. As above mentioned, when the membrane assembly 13 is mounted to the first end 121 of the main body 120 of the second culture tank 12, the membrane assembly 13 may substantially seal the first opening 1210 and the surface 1332 of the membrane 133 of the membrane assembly 13 may face the inner space 1200 of the main body 120 of the second culture tank 12. Moreover, the first group of cells 71 may be retained on the surface 1331 of the membrane 133 of the membrane assembly 13.

Referring to FIG. 12E, the second culture tank 12 with the membrane assembly 13 is received in one of wells 60 of the second culture plate 6. In some embodiments of the present disclosure, the six second culture tanks 12 equipped with membrane assemblies 13 may be placed individually in the six wells 60 of the second culture plate 6. In addition, the well 60 may be filled with cell culture medium 65.

Referring to FIG. 12F, a second group of cells 72 is cultivated in the second culture tank 12. Referring to FIG. 12F, the main body 120 of the second culture tank 12 is inserted into the well 60 of the second culture plate 6. The first end 121 of the main body 120 of the second culture tank 12 is submerged in the cell culture medium 65, causing the entire membrane assembly 13 to also be submerged in the cell culture medium 65. The second group of cells may be cultivated in the inner space 1200 of the main body 120 of the second culture tank 12. The second group of cells 72 may be grown on the surface 1333 of the membrane 133 of the membrane assembly 13. Meanwhile, the first group of cells 71 retained on the surface 1331 of the membrane 133 of the membrane assembly 13 may be cultivated in the inner space 600 of the well 60 of the second culture plate 6. That is, the first group of cells 71 and the second group of cells 72 may be cultivated in a horizontal state. In some embodiments of the present disclosure, the second group of cells 72 includes one or more types of cells. In some embodiments of the present disclosure, the cell type of the second group of cells 72 may be the same as or different from the cell type of the first group of cells 71.

Moreover, the trans-epithelial electrical resistance (TEER) may be monitored during the culture process. As shown in FIG. 12F, the electrodes 67, 68 may be respectively arranged in the inner space 1200 of the main body 120 of the second culture tank 12 and the inner space 600 of the well 60 of the second culture plate 6 so as to measure an electrical resistance across the membrane assembly 13.

In some embodiments of the present disclosure, a third group of cells 73 may be cultivated in the inner space 600 of the well 60 of the second culture plate 6. As shown in FIG. 12G, the third group of cells 73 may be grown on the bottom 601 of the inner space 600 of the well 60. That is, the first group of cells 71, the second group of cells 72 and the third group of cells 73 may be cultivated in a horizontal state. In some embodiments of the present disclosure, the third group of cells 73 includes one or more types of cells. In some embodiments of the present disclosure, the cell type of the third group of cells 73 may be the same as or different from the cell type of the first group of cells 71 and/or the cell type of the second group of cells 72.

Referring to FIG. 12H, after the second group of cells 72 has grown to cover the surface 1332 of the membrane 133 of the membrane assembly 13, the second culture tank 12 may be removed from the well 60 of the second culture plate 6, and the membrane assembly 13 may be detached from the first end 121 of the main body 120 of the second culture tank 12. In some embodiments of the present disclosure, the membrane assembly 13 is detached from the first end 121 of the main body 120 of the second culture tank 12 using the tool 4 and/or the method shown in FIG. 9A, FIG. 9B, and FIG. 9C.

The present disclosure provides a dual-sided multi-cell co-culture method and system, which addresses significant limitations in traditional single-layer cell culture systems. Here are the key technical characteristics and their corresponding benefits:
- Dual-Sided Cultivation Capability:
   Technical Characteristic: The present disclosure allows for the simultaneous cultivation of multiple cell types on both sides of a transparent porous membrane. This membrane is treated with plasma to graft biopolymers such as gamma-PGA, hyaluronic acid, collagen, chitin, chitosan, and fibroin.

Benefit: This dual-sided approach provides the ability to simulate more complex biological environments and interactions between different cell types, making it suitable for advanced tissue engineering and in vitro modeling of tissues like skin, blood vessels, and brain barriers.
- Three-Cell Group Co-Culturing:
   Technical Characteristic: The system can cultivate up to three distinct groups of cells, each potentially comprising multiple cell types, in a horizontal arrangement.

Benefit: This feature enhances the simulation of physiological conditions by allowing for the creation of multi-layered tissue structures, such as endothelial and epithelial tissues, which are essential for realistic in vitro models for drug testing, disease modeling, and tissue regeneration studies.
- Real-Time TEER Monitoring:
   Technical Characteristic: The present system may be equipped with electrodes that enable the real-time monitoring of Trans-Epithelial Electric Resistance (TEER) during the cell culture process.

Benefit: Continuous TEER measurement ensures the integrity of cell monolayers and provides crucial data on barrier function, which is vital for studies involving epithelial and endothelial barrier properties, as well as for assessing the effects of drugs and other compounds on cell layers.
- Customizable Membrane Stiffness:
   Technical Characteristic: The membrane's stiffness can be adjusted by varying the types and concentrations of grafted biopolymers, tailored to the specific requirements of different cell types.

Benefit: This adaptability allows researchers to optimize the mechanical properties of the cell culture environment to match the native tissues, enhancing cell growth and function, and improving the accuracy of experimental results.
- Scalable and Modular Design:
   Technical Characteristic: The apparatus features a modular design with detachable culture tanks and membrane assemblies, allowing for easy assembly, disassembly, and customization of the culture setup.

Benefit: The modularity and scalability of the system facilitate high-throughput screening and large-scale studies, making it a versatile tool for both research and industrial applications in biotechnology and pharmaceuticals.
- Versatile Application:
   Technical Characteristic: The present disclosure can simulate various physiological environments, including transdermal absorption, general blood vessel conditions, and brain blood vessels.

Benefit: This versatility broadens the application scope of the system, making it suitable for a wide range of biomedical research areas, including drug delivery, toxicology, and tissue engineering.

In summary, the dual-sided multi-cell co-culture method and apparatus offer significant advancements over traditional single-layer culture systems. Its ability to cultivate multiple cell types in a controlled, physiologically relevant environment, along with real-time monitoring and customizable features, positions it as a powerful tool for advancing research and development in the fields of medicine and biotechnology.

As used herein, the singular terms "a," "an," and "the" may include a plurality of referents unless the context clearly dictates otherwise.

As used herein, the terms "approximately," "substantially," "substantial" and "about" are used to describe and account for small variations. When used in conjunction with an event or circumstance, the terms can refer to instances in which the event or circumstance occurs precisely as well as instances in which the event or circumstance occurs to a close approximation. For example, when used in conjunction with a numerical value, the terms can refer to a range of variation of less than or equal to ±10% of that numerical value, such as less than or equal to ±5%, less than or equal to ±4%, less than or equal to ±3%, less than or equal to ±2%, less than or equal to ±1%, less than or equal to ±0.5%, less than or equal to ±0.1%, or less than or equal to ±0.05%. For example, two numerical values can be deemed to be "substantially" the same or equal if the difference between the values is less than or equal to ±10% of an average of the values, such as less than or equal to ±5%, less than or equal to ±4%, less than or equal to ±3%, less than or equal to ±2%, less than or equal to ±1%, less than or equal to ±0.5%, less than or equal to ±0.1%, or less than or equal to ±0.05%. For example, "substantially" parallel can refer to a range of angular variation relative to 0° that is less than or equal to ±10°, such as less than or equal to ±5°, less than or equal to ±4°, less than or equal to ±3°, less than or equal to ±2°, less than or equal to ±1°, less than or equal to ±0.5°, less than or equal to ±0.1°, or less than or equal to ±0.05°. For example, "substantially" perpendicular can refer to a range of angular variation relative to 90° that is less than or equal to ±10°, such as less than or equal to ±5°, less than or equal to ±4°, less than or equal to ±3°, less than or equal to ±2°, less than or equal to ±1°, less than or equal to ±0.5°, less than or equal to ±0.1°, or less than or equal to ±0.05°.

Additionally, amounts, ratios, and other numerical values are sometimes presented herein in a range format. It is to be understood that such range format is used for convenience and brevity and should be understood flexibly to include numerical values explicitly specified as limits of a range, but also to include all individual numerical values or sub-ranges encompassed within that range as if each numerical value and sub-range were explicitly specified.

While the present disclosure has been described and illustrated with reference to specific embodiments thereof, these descriptions and illustrations do not limit the present disclosure. It should be understood by those skilled in the art that various changes may be made and equivalents may be substituted without departing from the true spirit and scope of the present disclosure as defined by the appended claims. The illustrations may not be necessarily drawn to scale. There may be distinctions between the artistic renditions in the present disclosure and the actual apparatus due to manufacturing processes and tolerances. There may be other embodiments of the present disclosure which are not specifically illustrated. The specification and drawings are to be regarded as illustrative rather than restrictive. Modifications may be made to adapt a particular situation, material, composition of matter, method, or process to the objective, spirit and scope of the present disclosure. All such modifications are intended to be within the scope of the claims appended hereto. While the methods disclosed herein are described with reference to particular operations performed in a particular order, it will be understood that these operations may be combined, sub-divided, or re-ordered to form an equivalent method without departing from the teachings of the present disclosure. Accordingly, unless specifically indicated herein, the order and grouping of the operations are not limitations on the present disclosure.

## Claims

1. A dual-sided multi-cell co-culture apparatus (1), comprising:
a first culture tank (11); comprising:
a main body (110) being substantially cylindrical and having a first end (111) and a second end (112) opposite to the first end (111);
a first opening (1110) at the first end (111) of the main body (110); and
a second opening (1120) at the second end (112) of the main body (110);
a second culture tank (12); and
a main body (120) being substantially cylindrical and having a first end (121) and a second end (122) opposite to the first end (121);
a first opening (1210) at the first end (121) of the main body (120); and
a second (1220) opening at the second end (122) of the main body (120);
a membrane assembly (13);
wherein the membrane assembly (13) is configured to be removably mounted at the first end (111) of the main body (110) of the first culture tank (11), and wherein, when the membrane assembly (13) is mounted at the first end (111) of the main body (110) of the first culture tank (11), the membrane assembly (13) is configured to substantially seal the first opening (1110) of the main body (110) of the first culture tank (11);
wherein the membrane assembly (13) is configured to be removably mounted at the first end (121) of the main body (120) of the second culture tank (12), and wherein, when the membrane assembly (13) is mounted at the first end (121) of the main body (120) of the second culture tank (12), the membrane assembly (13) is configured to substantially seal the first opening (1210) of the main body (120) of the second culture tank (12).

2. The dual-sided multi-cell co-culture apparatus of claim 1, wherein the membrane assembly (13) comprises:
a ring (131), which is configured to match a periphery of the first opening (1110) of the main body (110) of the first culture tank (11) and configured to match a periphery of the first opening (1210) of the main body (120) of the second culture tank (12); and
a membrane (133), which attached to the ring (131).

3. The dual-sided multi-cell co-culture apparatus of claim 2, wherein the membrane (133) comprises a transparent porous membrane, and wherein one or more biopolymers are grafted onto both opposing surfaces of the transparent porous membrane.

4. The dual-sided multi-cell co-culture apparatus of claim 3, wherein the transparent porous membrane is made of a material selected from a group consisting of PET, PC, PTFE, and PVDF.

5. The dual-sided multi-cell co-culture apparatus of claim 3, wherein the transparent porous membrane has a pore size selected from a group consisting of 400 nm, 1 micron, 3 microns, and 8 microns.

6. The dual-sided multi-cell co-culture apparatus of claim 3, wherein the one or more biopolymers are selected a group consisting of gamma-PGA, hyaluronic acid, collagen, chitin, chitosan, fibroin, and poly-L-lysine.

7. The dual-sided multi-cell co-culture apparatus of claim 2, wherein the membrane (133) substantially covers a first side (1312) of the ring (131), and wherein the membrane has a first surface (1331) that is recessed with respect to a second side (1311) of the ring (131), the second side (1311) being opposite to the first side (1312) of the ring.

8. The dual-sided multi-cell co-culture apparatus of claim 7, wherein, when the membrane assembly (13) is mounted in the first opening (1110) of the main body (110) of the first culture tank (11), the first surface (1331) of the membrane (133) faces an inner space (1100) of the main body (110) of the first culture tank (11), and wherein, when the membrane assembly (13) is mounted in the first opening (1210) of the main body (120) of the second culture tank (12), the first surface (1331) of the membrane (133) faces away from an inner space (1200) of the main body (120) of the second culture tank (12).

9. The dual-sided multi-cell co-culture apparatus of claim 1, wherein the first culture tank (11) is configured to be received in a well (50) of a first culture plate (5), and wherein the second culture tank (12) is configured to be received in a well (60) of a second culture plate (6).

10. The dual-sided multi-cell co-culture apparatus of clam 9, wherein, when the first culture tank (11) is mounted with the membrane assembly (13) and received in the well (50) of the first culture plate (5), the membrane assembly (13) is configured to separate an inner space (1100) of the first culture tank (11) and an inner space (500) of the well (50) of the first culture plate (5); and wherein, when the second culture tank (12) is mounted with the membrane assembly (13) and received in the well (60) of the second culture plate (6), the membrane assembly (13) is configured to separate an inner space (1200) of the second culture tank (12) and an inner space (600) of the well (60) of the second culture plate (6).

11. The dual-sided multi-cell co-culture apparatus of clam 9, wherein, when the first culture tank (11) is mounted with the membrane assembly (13) and received in the well (50) of the first culture plate (5), a first surface (1331) of a membrane (13) of the membrane assembly (13) is configured to cultivate a first group (71) of cells.

12. The dual-sided multi-cell co-culture apparatus of clam 11, wherein, when the second culture tank (12) is mounted with the membrane assembly (13) and received in the well (60) of the second culture plate (6), a second surface (1332) of the membrane (133) the membrane assembly (13) is configured to cultivate a second group (72) of cells, the second surface (1332) opposite to the first surface (1331).

13. The dual-sided multi-cell co-culture system of claim 12, wherein a bottom of the well (60) of the second culture plate (6) is configured to cultivate a third group (73) of cells.

14. A method of co-culturing cells, comprises:
equipping a membrane assembly (13) with a first culture tank (11), wherein the membrane assembly (13) has a first surface (1331) facing an inner space (1100) of the first culture tank (11);
arranging the first culture tank (11) with the membrane assembly (13) in a well (50) of a first culture plate (5);
cultivating a first group (71) of cells on the first surface (1331) of the membrane assembly (13);
removing the first culture tank (11) with the membrane assembly (13) from the well (50) of the first culture plate (5);
separating the membrane assembly (13) from the first culture tank;
equipping the membrane assembly (13) with a second culture tank (13), wherein the membrane assembly has a second surface (1332), which is opposite to the first surface (1331), facing an inner space (1200) of the second culture tank (12);
arranging the second culture tank (12) with the membrane assembly (13) in a well (60) of a second culture plate (6); and
cultivating a second group of cells (72) on the second surface (1332) of the membrane assembly (13).

15. The method of claim 14, further comprising: cultivating a third group of cells (73) on a bottom surface (601) of the well (60) the second culture plate (6) while cultivating the second group of cells (72) on the second surface (1332) of the membrane assembly (13).
